# EUROPEAN PATENT SPECIFICATION

(11) **EP 1 013 641 B1**
(45) Date of publication and mention of the grant of the patent: **24.11.2004**
(21) Application number: 99123664.7
(22) Date of filing: 29.11.1999
(51) Int. Cl.: C07C 309/65, C07C 381/00, C07C 45/68, C07C 49/80

(54) **Acylation agent**
Acylierungsmittel
Agent d'acylation

(30) Priority: 23.12.1998 US 113869 P
(43) Date of publication of application: 28.06.2000
(73) Proprietor: Haldor Topsoe A/S, 2800 Lyngby (DK)
(72) Inventor: Schiodt, Niels Christian, 2700 Bronshoj (DK)
(74) Representative: Grünecker, Kinkeldey, Stockmair & Schwanhäusser Anwaltssozietät

(56) References cited:
- F. EFFENBERGER, ET AL.: "Trifluoromethanesulphonic acid-carboxylic anhydrides, highly active acylating agents" ANGEWANDTE CHEMIE, INTERNATIONAL EDITION, vol. 11, no. 4, April 1972 (1972-04), pages 299-300, XP002225247 Verlag Chemie, Weinheim, DE ISSN: 0570-0833
- M.H. KARGER, ET AL.: "Mixed sulphonic-carboxylic anhydrides. III. Reactions with aromatic ethers and aromatic hydrocarbons" JOURNAL OF ORGANIC CHEMISTRY, vol. 36, no. 4, 26 February 1971 (1971-02-26), pages 540-544, XP002225248 American Chemical Society, Washington, DC, US ISSN: 0022-3263
- F. EFFENBERGER, ET AL.: "The first unequivocal evidence of the reacting electrophile in aromatic acylation reactions" JOURNAL OF THE AMERICAN CHEMICAL SOCIETY, vol. 118, no. 11, 18 December 1996 (1996-12-18), pages 12572-12579, XP002225249 American Chemical Society, Washington, DC, US ISSN: 0002-7863

## Description

The invention relates to an acylation agent and more particularly to 1,1,1-tris halo acetyl trifluoromethanesulphonate acylation compounds and a method of preparing these compounds.

### BACKGROUND OF THE INVENTION

Acylation of aromatic molecules to produce aromatic ketones is a frequently employed reaction in research as well as in the industry. Such acylation reactions are usually carried out with acyl halides or acid anhydrides under so-called Friedel-Crafts conditions, which imply the use of a Lewis acid catalyst, in particular anhydrous aluminum chloride. In some applications, however, such Lewis acid catalysts will lead to undesired side-reactions. If the desired product contains halo-alkyl groups in a position β or γ to the carbonyl group, acylation of the aromatic molecule with the corresponding β or γ haloalkyl acyl halide or the corresponding β or γ haloalkyl acid anhydride will cause ring closure of the product under Friedel-Crafts conditions. This is due to the fact that Friedel-Crafts catalysts catalyse not only acylation, but also alkylation of aromatic molecules (from halo-alkyl reagents) and to the fact that intramolecular reactions leading to the formation of 5 or 6 membered rings are proceeding fast.

This invention provides a reliable synthetic paths through which a 1,1,1-tris(halomethyl)acetyl group is covalently bound to an aromatic molecule. In the known methods treating 1,1,1-tris(halomethyl)acetyl chloride with AlCl₃ in the presence of an aromatic molecule as e.g. benzene would inevitably give rise to the bicyclic compound 2,2-bis(chloromethyl)-1-indanone for the reasons outlined above.

Mixed carboxylic-trifluoromethanesulphonic anhydrides are strong acylating reagents, but usually rather unstable when derived from aliphatic carboxylic acids (F. Eftenberger, and G. Epple, Angew. Chem. 84, 294, 1972). We found, however, that tris(chloromethyl)acetyl-trifluoromethanesulphonate is fairly stable in solution, yet very reactive toward aromatic molecules with respect to acylation by electrophilic aromatic substitution.

### SUMMARY OF THE INVENTION

The invention provides a new compound 1,1,1-tris(halomethyl)acetyl trifluoromethanesulphonate and a method for preparing these compounds.

Use of these compounds as acylating reagent is shown in the reaction scheme:

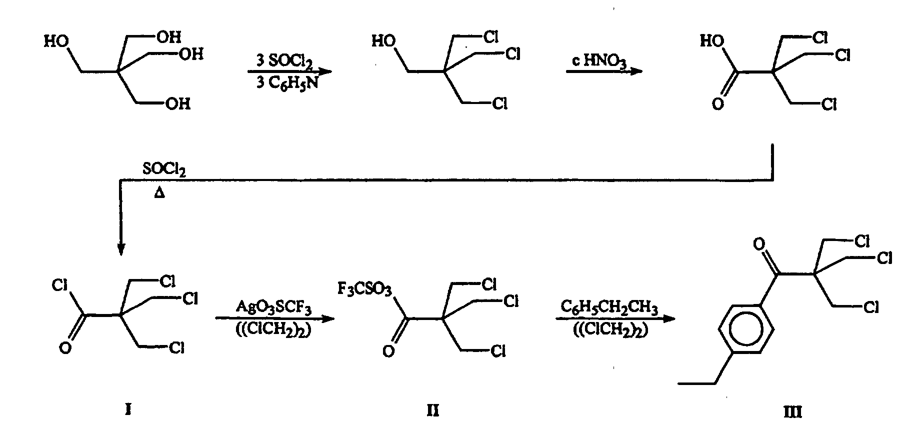

### Examples

The synthesis of tris(chloromethyl)acetyl chloride ((ClCH₂)₃CCOCl) **I** from pentaerythrithol is described in the literature (A. Mooradian and J. B. Cloke, J. Chem. Soc. 67, 942, 1945). ¹H-NMR and ¹³C-NMR spectra were recorded for all intermediates and found to be in accordance with the proposed structures.

### Example 1

### Preparation of 1,1,1-tris(chloromethyl)acetyl trifluoromethanesulphonate.

Tris(chloromethyl)acetyl chloride (ClCH₂)₃CCOCl (56 g; 0.25 mol) were dissolved in 1,2-dichloroethane (200 ml) and stirred magnetically with solid silver trifluoromethanesulfonate (64.25 g; 0.25 mole) at room temperature for 24 hours in a dry atmosphere in the dark. This caused the formation of an almost quantitative precipitate of silver chloride, which was filtered off in a dry atmosphere and with dry equipment. The filtrate-containing the mixed carboxylic acid-trifluoromethanesulphonic acid anhydride was used immediately for acylation reactions.

### Example 2

### Preparation of III and IV.

To the filtrate was added ethylbenzene (66 ml; 0.27 mole) and the mixture was refluxed for 4 hours in the dark and in a dry atmosphere. The solution was cooled to room temperature and washed consecutively with aqueous sodium hydrogen carbonate, water and brine. The solvent was evaporated on a rotary evaporator, leaving a brown oil. This was redissolved in dichloromethane (100 ml) and decolorised by running through a short column of silica gel (4 x 10 cm). The column was eluted with a further 300 ml dichloromethane and all the fractions combined. After removal of the solvent, the raw product was dissolved in 300 ml boiling petrol ether (60-80°C) and cooled to -18°C overnight causing the crystallisation of the product III. By this procedure, a yield of 41,9 g (57% based on I) of pure III was obtained. The structure was identified by 1H- and ¹³C-NMR spectroscopy.

The mother liqour contained some additional III, but mainly the ortho-isomer 2-ethyl-(1,1,1-tris(chloromethyl)acetyl benzene IV, which was identified spectroscopically. The reaction produces the para-isomers and ortho-isomers in a ratio of approximately 2:1.

## Claims

1. Compounds having the general formula CF₃SO₃C(O)C(CH₂X)₃, where X is selected from fluorine, chlorine, bromine and iodine.

2. The compounds of claim 1, wherein X is selected from chlorine, bromine and iodine.

3. The compounds of claim 1, wherein X is chlorine.

4. Method of preparing a compound as claimed in claim 1, comprising steps of reacting a tris(halomethyl) acetyl halide with trifluoromethanesulphonic acid or salts thereof.

5. Use of the compounds of claim 1 as acylation agents.

## Patentansprüche

1. Verbindungen mit der allgemeinen Formel CF₃SO₃C(O)C(CH₂X)₃, wobei X ausgewählt ist aus Fluor, Chlor, Brom und lod.

2. Verbindungen nach Anspruch 1, wobei X ausgewählt ist aus Chlor, Brom und lod.

3. Verbindungen nach Anspruch 1, wobei X Chlor ist.

4. Verfahren zum Herstellen einer Verbindung wie in Anspruch 1 beansprucht, umfassend Schritte des Umsetzens eines Tris(halogenmethyl)acetylhalogenids mit Trifluormethansulfonsäure oder Salzen davon.

5. Verwendung der Verbindungen nach Anspruch 1 als Acylierungsmittel.

## Revendications

1. Composés de formule générale CF₃SO₃C(O)C(CH₂X)₃, dans laquelle X est choisi parmi le fluor, le chlore, le brome et l'iode.

2. Composés selon la revendication 1, dans lesquels X est choisi parmi le chlore, le brome et l'iode.

3. Composés selon la revendication 1, dans lesquels X est le chlore.

4. Procédé de préparation d'un composé selon la revendication 1, comprenant les étapes de réaction d'un halogénure de tris(halogénométhyl)acétyle avec de l'acide trifluorométhanesulfonique ou des sels de celui-ci.

5. Utilisation des composés selon la revendication 1 en tant qu'agents d'acylation.
